(19) Europäisches Patentamt
European Patent Office
Office européen des brevets

(11) EP 3 352 812 B1

(12) EUROPÄISCHE PATENTSCHRIFT

(45) Veröffentlichungstag und Bekanntmachung des Hinweises auf die Patenterteilung:
03.07.2019 Patentblatt 2019/27

(51) Int Cl.:
A61M 1/36 (2006.01)  A61B 5/145 (2006.01)
A61B 5/1455 (2006.01)  G01N 33/49 (2006.01)

(21) Anmeldenummer: 16770018.6

(22) Anmeldetag: 22.09.2016

(86) Internationale Anmeldenummer:
PCT/EP2016/072548

(87) Internationale Veröffentlichungsnummer:
WO 2017/050899 (30.03.2017 Gazette 2017/13)

(54) **BESTIMMUNG IN BLUT GELÖSTER GASE IM EXTRAKORPORALEN KREISLAUF**

DETERMINATION OF GASES DISSOLVED IN BLOOD IN THE EXTRACORPOREAL BLOOD FLOW

DÉTERMINATION DE GAZ DISSOUS DANS LE SANG DANS LA CIRCULATION EXTRACORPORELLE

(84) Benannte Vertragsstaaten:
AL AT BE BG CH CY CZ DE DK EE ES FI FR GB GR HR HU IE IS IT LI LT LU LV MC MK MT NL NO PL PT RO RS SE SI SK SM TR

(30) Priorität: 26.09.2015 DE 102015012519

(43) Veröffentlichungstag der Anmeldung:
01.08.2018 Patentblatt 2018/31

(73) Patentinhaber: Fresenius Medical Care Deutschland GmbH
61352 Bad Homburg (DE)

(72) Erfinder: MAIERHOFER, Andreas
97422 Schweinfurt (DE)

(74) Vertreter: Schön, Andrea
Fresenius Medical Care AG & Co. KGaA
Global Intellectual Property
Frankfurter Straße 6-8
66606 St. Wendel (DE)

(56) Entgegenhaltungen:
WO-A1-2014/049458    DE-A1- 3 222 617
US-A- 4 640 820      US-A- 4 717 548

**Beschreibung**

**Technisches Gebiet**

Die Erfindung betrifft eine Blutreinigungsvorrichtung mit einem extrakorporalen Blutkreislauf, einem Gassensor und einer Auswerteeinheit, wodurch der Partialdruck eines Gases im extrakorporalen Blutkreislauf bestimmt und überwacht werden kann, sowie ein Verfahren zur Bestimmung des Partialdrucks eines Gases in Blut.

**Hintergrund**

Bei der Durchführung von Blutreinigungsbehandlungen, die mit einem extrakorporalen Blutkreislauf einhergehen, ist die Überwachung der Patientenparameter, insbesondere der Vitalparameter, von großer Wichtigkeit.

In Dialysekliniken erfolgt dies typischerweise durch regelmäßige Beobachtung des Patienten durch das Pflegepersonal. Bei Langzeitdialysen über Nacht oder in der Heimdialyse ist eine Überwachung durch Pflegepersonal nur eingeschränkt oder gar nicht vorhanden.

Weitere Möglichkeiten ergeben sich durch automatisierte Überwachungen, die selbständig durch das Dialysegerät durchgeführt werden. Beispiele dafür sind Blutdruckmessungen, Elektrokardiogramme und die Überwachung mit Pulsoximetern. Für diese Messungen ist allerdings die Verwendung zusätzlicher Sensoren notwendig, was bei der Heimdialyse oft schwierig und oft auch störend für den Schlaf des Patienten ist.

Wird zum Monitoring eines Vitalparameters die Bestimmung des Partialdrucks eines Gases in Blut angewendet, erfolgt dies meist durch Messungen in der Atemluft des Patienten, wohin diese Substanzen durch den Gasaustausch in den Alveolen gelangen ("Potential Application of Exhaled Breath Monitoring in Renal Replacement Therapy", Kelly Paschke et al., Poster Presentation ASN 2013).

Beispiele für Blutgase, die relevante Informationen für das Monitoring von Patienten liefern können, sind Kohlendioxid, Aceton und Ammonium.

Die Bestimmung von Aceton kann z.B. bei der Behandlung von Diabetes-Patienten hilfreiche Daten liefern, da so eine Ketoazidose diagnostiziert werden kann. Die Aufzeichnung der Werte über mehrere Behandlungen kann hilfreich bei der Dosierung von Insulin bei Diabetikern in der Dialyse sein.

Die Bestimmung der Ammoniumkonzentration im Atem steht in Zusammenhang mit der Harnstoffkonzentration in Blut ("correlation of breath ammonia with blood urea nitrogen and creatinine during hemodialysis, Narashimhan et al. PNAS Vol.98, S. 4617ff). Daher sind mittels eines Ammoniumsensors praktisch alle Analysen möglich, die sonst auf der Bestimmung der Harnstoffkonzentration in Blut beruhen.

Von besonderem Interesse und auch am weitesten verbreitet ist dabei die Überwachung des CO2-Partialdrucks des Blutes, welcher in direktem Zusammenhang mit der Atmung bzw. der Sauerstoffsättigung steht und damit einen wichtigen Vitalparameter darstellt. Ein Anstieg des CO2-Partialdrucks kann auf eine unzureichende oder sogar fehlende Atmung, wie z.B. bei der Schlaf-Apnoe, hindeuten. Betroffen von Schlaf-Apnoe sind vor allem Menschen, die älter und/oder übergewichtig sind (ggf. auch mit anderen neurologischen Störungen). Die Prävalenz wird in den USA aus 18 Millionen Patienten geschätzt. Dialysepatienten sind aufgrund ihrer Demographie und der für sie typischen Comorbiditäten dabei überproportional betroffen. Bei einer ESRD-Prävalenz von ca. 600 ppm und einer angenommenen erhöhten Schlaf-Apnoe-Prävalenz um den Faktor 2 gegenüber der Normalbevölkerung wären also in den USA allein >2000 Dialysepatienten von Schlafapnoe betroffen. Schlaf-Apnoen führen zu einer verminderten nächtlichen Erholung und können zu Herz-Kreislauferkrankungen wie Bluthochdruck, Rechtsherzinsuffizienz bis hin zum plötzlichen Herztod führen.

Bei dem herkömmlichen Apnoe-Monitoring wird z.B. die Messung des $CO_2$-Gehalts der Atemluft verwendet. Dazu kann die direkte Bestimmung des $CO_2$-Gehalts der Atemluft durch Analyse der über eine Atemmaske strömenden Luft oder der in einen in die Nase eingeführten Schlauch strömenden Luft (End Tidal $CO_2$-Monitoring = $EtCO_2$-Monitoring) erfolgen. Alternativ ist die Bestimmung in Blut durch transcutane $CO_2$ Messungen oder auch die Blutgasanalyse anhand von Blutproben anwendbar. All diese Verfahren erfordern zusätzliche Apparaturen, am Patienten fixierte Sensoren und besonders geschultes Personal. Sie sind mit Belastungen für den Patienten, z.B. Dialysepatienten, verbunden, die von eingeschränktem Komfort durch die Fixierung zusätzlicher Sensorik und möglichen Hautirritationen durch das Heizen des Sensors beim transcutanen $CO_2$-Monitoring bis hin zu Blutentnahmen für die Blutgasanalyse gehen. Die nichtinva- siven Methoden des $CO_2$-Monitorings sind zudem auch störanfällig. Während beim $EtCO_2$ Monitoring insbesondere eine Mundatmung anstelle einer Atmung durch die Nase das Messergebnis verfälschen kann, führt beim transcutanen $CO_2$-Monitoring eine mangelnde Durchblutung der unter dem Sensor liegenden Hautpartie zu fehlerhaften Ergebnissen.

Eine weitere Anwendung der $CO_2$-Konzentrations-Bestimmung speziell bei Dialysepatienten ist die Kontrolle der pH-Status des Blutes zur Erkennung einer Azidose.

So wird in der WO 2013/156435 eine Vorrichtung zur extrakorporalen Blutbehandlung beschrieben, die durch Messung der Bicarbonatkonzentration und des pH-Wertes in Blut direkt den $CO_2$-Partialdruck bestimmt. Hier werden die Daten zur Einstellung des pH-Wertes des Blutes durch Regelung der Bicarbonatkonzentration im Dialysat in Abhängigkeit von den erhaltenen Messdaten verwendet. Hier ist ein direkter Kontakt des Sensors mit dem Patientenblut erforderlich, was das Risiko der Kreuzkontamination mit sich bringt.

Der vorliegenden Erfindung liegt daher die Aufgabe zugrunde, eine sichere, automatische und kontinuierliche Überwachung eines Patientenparameters, insbesondere des $CO_2$-Partialdrucks des Blutes, zu gewährleisten, ohne dass zusätzliche Gebrauchsmaterialien benötigt werden oder zusätzliche Belastungen für den Patienten entstehen.

**Zusammenfassung der Erfindung**

Diese Aufgabe wird gelöst durch eine Blutbehandlungsvorrichtung gemäß Anspruch 1 und einem Verfahren gemäß Anspruch 15. Besondere Ausführungsformen der Erfindung sind Gegenstand der abhängigen Ansprüche.

In einer Ausführungsform weist eine Blutbehandlungsvorrichtung mit einem extrakorporalen Blutkreislauf die standardmäßigen Komponenten eines Blutschlauchsystems auf. Dieses Blutschlauchsystem umfasst eine erste Leitung, die mit einem ersten Ende mit dem Patienten zur Blutentnahme verbindbar ist und mit einem zweiten Ende mit der Ausgleichskammer verbunden ist. In dieser ersten Leitung ist eine erste Blutpumpe angeordnet, die das vom Patienten entnommene Blut im extrakorporalen Kreislauf in die Ausgleichskammer fördert. In dieser Ausgleichskammer entsteht dann ein Blutpegel, wodurch sich ein Kontakt des Blutes zu einem sich oberhalb des Blutpegels befindlichen Gasraum bildet. Aus der Ausgleichskammer wird das Blut durch eine zweite Leitung zu der Blutreinigungseinheit geführt. Weiterhin weist die erfindungsgemäße Blutbehandlungsvorrichtung zumindest einen mit der Ausgleichskammer verbindbaren Gassensor zur Messung der Konzentration eines Gases in dem Gasraum der Ausgleichskammer auf. Es ist davon auszugehen, dass sich in dem Gasraum oberhalb des Blutpegels in einer Ausgleichskammer eines extrakorporalen Kreislaufs ähnliche Gleichgewichtskonzentrationen einstellen wie in den Alveolen der Lunge. Weiterhin umfasst die Blutbehandlungsvorrichtung auch eine Auswerteeinheit zur Auslesung und Auswertung der Messdaten des Gassensors.

Vorteilhaft ist eine Positionierung des Gassensors, die eine kontaktlose, weitgehend wartungsfreie und vor Kreuzkontamination sichere Messung ermöglicht. Weiterhin vorteilhaft ist, dass die standardmäßig bei der Blutbehandlung verwendeten Verbrauchsmaterialien für das extrakorporale Schlauchsystem eingesetzt werden können.

Abhängig von dem Parameter bzw. den Parametern, die überwacht werden sollen, kann als Gassensor ein Sensor für Kohlendioxid oder Ammonium oder Aceton eingesetzt werden. Es ist auch denkbar, verschiedene Gassensoren zu kombinieren.

Gassensoren z.B. für Kohlendioxid für den relevanten Konzentrationsbereich sind kommerziell erhältlich und basieren z.B. auf einer Bestimmung der spezifischen Adsorption von Strahlung im Infrarotbereich. Diese Sensoren sind bis zu 100% relative Feuchte unter nicht kondensierbaren Bedingungen einsetzbar.

Zur Vermeidung einer Kondensation auf den Gassensoren können diese beheizbar ausgelegt sein.

Da bei der Auswertung der Messdaten die Berücksichtigung der Bluttemperatur zu einem genaueren Ergebnis führt, kann in dem extrakorporalen Kreislauf z.B. in der Zuleitung, der Ableitung oder auch in der Ausgleichskammer ein Temperatursensor vorgesehen sein, der die Messergebnisse an die Auswerteeinheit liefert, welche diese bei der Auswertung der Messdaten des Gassensors berücksichtigt.

Ein weiterer Parameter, der bei der Auswertung der Messdaten zu einer Verbesserung der Genauigkeit des Messergebnisses führen kann, ist der aktuell am Gassensor vorliegende Druck. Eine erfindungsgemäße Ausführungsform der Blutbehandlungsvorrichtung kann einen Drucksensor aufweisen, der den Druck im Gasraum, in dem der Gassensor angeordnet ist, messen kann, und der die Messergebnisse an die Auswerteeinheit liefert, welche diese bei der Auswertung der Messdaten des Gassensors berücksichtigt.

Während die Ausgleichskammer vorteilhaft ein Teil des Blutschlauchsystems ist, kann der Gassensor und die Auswerteeinheit in der Blutbehandlungsmaschine integriert sein. In einer alternativen Ausführungsform kann der Gassensor und die Auswerteeinheit als separates Modul ausgestaltet sein, das mit der Blutbehandlungseinrichtung verbunden werden kann. Die Verbindung der Ausgleichskammer mit dem Gassensor kann über eine Verbindungsleitung, insbesondere einen Verbindungsschlauch erfolgen. Zwischen dem Verbindungsschlauchs und dem Gassensor kann ein Hydrophobfilter vorgesehen sein, um eine Kontamination der Behandlungsmaschine durch Blut zu verhindern.

In Abhängigkeit von der Länge der Verbindungsleitung kann der Transport durch reine Diffusion zu langsam sein, so dass die sich in der Ausgleichskammer direkt über dem Blutpegel einstellende Gaskonzentration aktiv an den Gassensor transportiert werden muss. Dazu kann die erfindungsgemäße Blutbehandlungsvorrichtung Mittel zur Erzeugung einer Gasströmung aufweisen. Diese Mittel können über eine Flussrestriktion in der zweiten Leitung des extrakorporalen Blutkreislaufs einem Hub des Blutpegels in der Ausgleichskammer und damit eine Verschiebung des Gasvolumens aus der Ausgleichskammer bewirken oder alternativ als direktes Gasfördermittel vorgesehen sein.

Zur Verschiebung des Blutpegels in der Ausgleichkammer kann in dem extrakorporalen Kreislauf in der zweiten Leitung ein Ventil vorgesehen sein. Dieses Ventil kann von der Steuer- und Auswerteeinheit so angesteuert werden, dass es zu bestimmten Zeitpunkten in der zweiten Leitung des extrakorporalen Kreislaufs einen geringeren Blutfluss einstellt als in der ersten Leitung, so dass der Blutpegel in der Ausgleichskammer ansteigt, d.h. es wird ein Anstieg des Blutpegels erzeugt. Mit dem Anstieg des Blutpegels wird ein Gasvolumen aus der Ausgleichskammer zu dem Gassensor verschoben. Um sicherzustellen, dass die sich in der Ausgleichskammer eingestellte Konzentration auch den Gassensor erreicht, sollte das verdrängte Volumen vorzugsweise mindestens doppelt so groß sein wie das sich in der Verbindung zwischen der Ausgleichskammer und dem Gassensor befindliche Volumen. Bei den in Blutreinigungsverfahren üblicherweise eingesetzten Schlauchsystemen kann mit einem Anstieg des Blutpegels in der Ausgleichskammer mit einem Hub von circa 1-2 cm das Gasvolumen aus der Ausgleichskammer zu dem Gassensor transportiert werden.

Alternativ kann eine Verschiebung des Pegelstandes durch eine zweite Blutpumpe in der zweiten Leitung erfolgen, die von der Steuereinheit derart angesteuert wird, dass sie mit einer von der ersten, vor der Ausgleichkammer in der ersten Leitung des extrakorporalen Kreislaufs angeordneten Blutpumpe abweichenden Förderrate läuft. Ist die Förderrate der zweiten Blutpumpe geringer als die Förderrate der ersten Blutpumpe, kommt es zu einem Anstieg des Blutpegels in der Ausgleichskammer. Umgekehrt kommt es zu einer Absenkung des Blutpegels in der Ausgleichskammer, wenn die zweite Blutpumpe mit einer höheren Förderrate läuft als die erste Blutpumpe.

Um eine Erhöhung des Drucks an dem Gassensor oder der Ausgleichskammer bei einer Pegelanhebung zu vermeiden, kann die erfindungsgemäße Blutbehandlungsvorrichtung hinter dem Gassensor angeordnete Ausgleichsmittel, genauer Druckausgleichsmittel, aufweisen. Diese Ausgleichsmittel können z.B. Ventile sein, die sich gegenüber der Umgebung oder gegenüber einem Compliance-Gefäß öffnen. Durch die Öffnung wird im Gassensor ein Druckausgleich erreicht.

Die erfindungsgemäße Blutbehandlungsvorrichtung kann weiterhin einen Kompressor aufweisen, der durch Einlassen von Umgebungsluft eine Absenkung und damit Rückstellung des Blutpegels in der Ausgleichskammer bewirken kann.

Für eine erneute Messung der Gaskonzentration in der Ausgleichskammer kann der Blutpegel dann wieder angehoben werden.

Mit dieser Anordnung sind vorzugsweise intermittierende Messungen der Gaskonzentration möglich. Die Zeitintervalle können dabei genügend klein gewählt werden, so dass ein enges Screening des Konzentrationsverlaufs möglich ist.

Alternativ kann die erfindungsgemäße Blutbehandlungsvorrichtung ein Gasfördermittel, z.B. ein miniaturisierter Blasebalg, aufweisen, das aktiv eine kontinuierliche Gasströmung erzeugt. Dazu ist die Ausgleichskammer derart ausgestaltet, dass sie zusätzlich zu dem ersten Verbindungsschlauch durch einen weiteren Verbindungsschlauch als Rückführungsschlauch mit dem Gassensor verbunden ist. Die Steuereinheit der Blutbehandlungsvorrichtung ist dabei so ausgelegt, das Gasfördermittel so anzusteuern, dass eine zirkulierende Gasströmung erzeugt und eine kontinuierliche Messung der Gaskonzentration erfolgen kann.

In der Auswerteeinheit, die aus den Messdaten des Gassensors den Partialdruck des Gases im Blut ausrechnet, kann ein Sollbereich für die gemessene Gaskonzentration oder den Partialdruck des Gases in Blut hinterlegt sein. Treten bei der Messung der Gaskonzentration Werte außerhalb dieses Sollbereichs auf, so kann durch die Auswerteeinheit ein Signal ausgegeben werden.

Dieses Signal kann z.B. an eine Alarmeinrichtung weitergeleitet werden, die ein Alarmsignal ausgibt, wodurch weitere Maßnahmen ausgelöst werden.

Weiterhin können die Messdaten bei der Steuerung der Dialysatzusammensetzung verwendet werden, z.B. der Einstellung der Bicarbonatkonzentration in der Dialyselösung im Falle einer Dialysebehandlung.

Die Blutbehandlungsvorrichtung nach der vorliegenden Lehre kann nach dem Prinzip der Hämodialyse, der Hämofiltration oder der Hämodiafiltration oder der Plasmaphese arbeiten. Die verwendete Blutreinigungseinheit kann damit als Dialysefilter oder Hämofilter oder Plasmafilter ausgebildet sein.

Ein Verfahren nach der vorliegende Lehre bestimmt den Partialdruck eines Gases in Blut eines extrakorporalen Blutkreislaufs in einer Blutbehandlungsvorrichtung nach Anspruch 1 durch Messung der Gaskonzentration in der Ausgleichskammer mittels eines Gassensors und Berechnung des Partialdrucks in Blut, z. B. den $p_{CO2}$ in Blut, durch Auswertung der Messdaten des Gassensors mittels der Steuer- und Auswerteeinheit.

Um einen räumlichen Abstand zwischen der Ausgleichskammer und dem Gassensor zu überbrücken, kann die Herstellung einer Gasströmung von der Ausgleichskammer zu dem Gassensor vorgesehen sein.

Diese Gasströmung kann durch einen Hub des Blutpegels in der Ausgleichskammer hergestellt werden. Durch Anhebung des Blutpegels in der Ausgleichskammer wird eine Verdrängung des Gases in der Ausgleichskammer in die Verbindungsleitung zu dem Gassensor erreicht. Um sicherzustellen, dass die sich in der Ausgleichskammer eingestellte Konzentration den Gassensor erreicht, sollte das verdrängte Volumen vorzugsweise mindestens doppelt so groß sein wie das sich in dem Verbindungsschlauch befindliche Volumen. Dabei genügt bei den handelsüblichen Blutschlauchsystemen ein Hub von 1-2cm in der Ausgleichskammer um das in der Ausgleichskammer befindliche Gas zu dem Gassensor zu transportieren. Der Hub kann durch eine intermittierende Flussrestriktion im Blutkreislauf flussabwärts der Ausgleichskammer erfolgen. Dazu kann die Auswerte- und Steuereinheit ein in der zweiten Leitung angeordnetes Ventil so ansteuern, dass dieses teilweise oder auch kurzzeitig ganz schließt. Gleichzeitig können entsprechende Mittel zum Druckausgleich, wie z. B. ein Drosselventil oder ein Ventil zu einem Compliance-Gefäß, geöffnet werden, so dass am Gassensor kein Überdruck entsteht. Eine anschließende Absenkung des Pegels kann durch einen Druckstoß durch einen Kompressor rückgängig gemacht werden.

Alternativ kann eine Pegelverschiebung in der Ausgleichskammer mittels einer zweiten Blutpumpe in der zweiten Leitung, der Ableitung erreicht werden. Durch Ansteuerung der Auswerte- und Steuereinheit können für die erste und für die zweite Blutpumpe unterschiedliche Förderraten eingestellt werden. Fördert die erste Blutpumpe mit einer größeren Förderrate als die zweite Blutpumpe erfolgt eine Anhebung des Blutpegels in der Ausgleichskammer. Umgekehrt ergibt sich einer Förderrate der ersten Blutpumpe, die kleiner ist als die der zweiten Blutpumpe, eine Absenkung des Blutpegels in der Ausgleichskammer.

Die Gasströmung kann aber auch durch einen aktiven Transport des Gases durch ein Gasfördermittel erfolgen.

## Kurzbeschreibung der Zeichnungen

Figur 1: Schematische Darstellung einer ersten Ausführungsform der erfindungsgemäßen Blutbehandlungsvorrichtung.

Figur 2: Schematische Darstellung einer zweiten Ausführungsform der erfindungsgemäßen Blutbehandlungsvorrichtung.

Figur 3: Korrelation zwischen prädialytischem $p_{CO2}$ und Bicarbonat

Figur 4: Simulierter $p_{CO2}$ Verlauf während einer Langzeitdialyse mit Apnoen.

## Detaillierte Beschreibung eines Ausführungsbeispiels

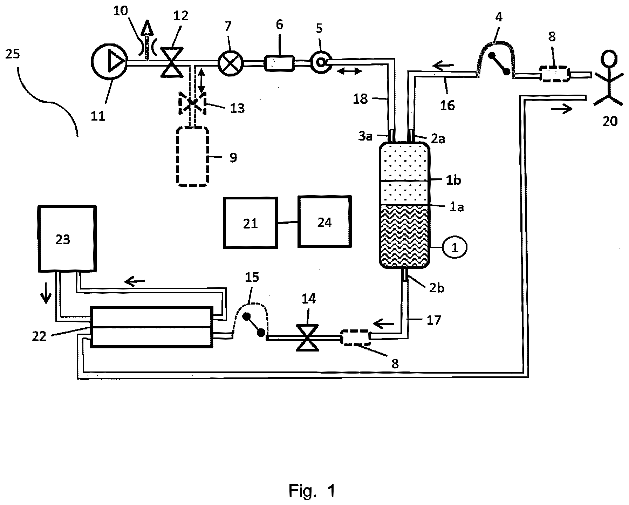

In Figur 1 ist eine erste Ausführungsform einer Blutbehandlungsvorrichtung (25) gemäß der vorliegenden Lehre gezeigt. Vom Patienten (20) wird Blut von der Blutpumpe (4) durch die erste Leitung (16), die arterielle Leitung, gefördert und gelangt über den Anschluss (2a) in die Ausgleichskammer (1). In der Ausgleichskammer (1) bildet sich ein Blutpegel (1a). Anschluss (2a) kann sich oberhalb oder unterhalb des Blutpegels (1a) befinden, d.h. z.B. auch benachbart zu Anschluss (2b), der einen Auslass aus der Ausgleichkammer bildet. Aus dem Auslass (2b) der Ausgleichskammer (1) fließt das Blut dann weiter über die zweite Leitung (17) zu der Blutreinigungseinheit (22), z.B. einem Dialysator und weiter zurück zu dem Patienten. Die Blutreinigungseinheit (22) ist dabei weiterhin mit einer Dialysatzubereitungseinheit (23) verbunden, in der die Dialyselösung mit der gewünschten Bicarbonatkonzentration hergestellt werden kann.

In der Ausgleichskammer (1) über dem Blutpegel stehen in Blut gelöste Gase im Gleichgewicht mit einer entsprechenden Gaskonzentration in dem Gasraum der Ausgleichskammer (1). Der Gasraum der Ausgleichskammer (1) ist über den Verbindungsschlauch (18) mit dem Gassensor (6) verbunden. Der Gassensor ist in dem festen Teil der Blutreinigungsvorrichtung, z.B. dem Dialysegerät, angeordnet. Zum Schutz des Gassensors vor einer Kontamination durch Blut, das bei Fehlfunktionen eventuell in den Verbindungschlauch aufsteigen kann, ist am Ende des Verbindungsschlauchs (18) ein Hydrophobfilter (5) vorgesehen. Hinter dem Hydrophobfilter wird das Gasgemisch zu einem $CO_2$-Sensor (6) geleitet. Dieser ist für einen Konzentrations-Messbereich von ca. 2 - 10 Vol% $CO_2$, entsprechend den physiologisch relevanten $CO_2$-Partialdrücken von 15-80mm Hg, optimiert.

Die von dem $CO_2$-Sensor aufgenommenen Messdaten werden von der Steuer- und Auswerteeinheit (21) ausgelesen und der $CO_2$-Partialdruck wird auf Grundlage der nachfolgend beschriebenen Formeln von der Steuer- und Auswerteeinheit berechnet.

Der Zusammenhang zwischen der $CO_2$-Konzentration $c_{CO2}$ in Blut und dem $CO_2$-Partialdruck pco2 im Gasraum wird durch das Henry'sche Gesetz beschrieben.

$$c_{CO_2} = K_{H,CO_2}\, p_{CO_2} \quad \textbf{Formel 1}$$

Die Henry'sche Konstante für $CO_2$ ist temperaturabhängig:

$$K_{H,CO_2}(T) = K^x_{H,CO_2} \exp\left\{-\frac{\Delta_s H}{R}\left(\frac{1}{T} - \frac{1}{T^x}\right)\right\} \quad \textbf{Formel 2}$$

mit $K^x = 3,4 \cdot 10^{-4} \frac{\text{mol}}{\text{m}^3 \text{Pa}}$, T$^x$=298,15 K und $\frac{\Delta_s H}{R} = 2400$ K.

Weiterhin steht die $CO_2$-Konzentration $c_{CO_2}$ in Blut gemäß der Henderson-Hasselbalch-Gleichung in Zusammenhang mit der Hydrogencarbonatkonzentration $c_{HCO3}$:

$$CO_{2,aq} + H_2O \leftrightarrow H^+ + HCO_3^-$$

$$pH = pK_s + \log\frac{c_{HCO_3^-}}{c_{CO_2}}$$

$$c_{CO_2} = c_{HCO_3^-} 10^{-(pH - pK_s)}$$

$$p_{CO_2}(T) = \frac{1}{K_{H,CO_2}(T)} c_{HCO_3^-} 10^{-(pH - pK_s)} \text{ mit } pKs \approx 6,1 \qquad \textbf{Formel 3}$$

Der sich im Gasraum über dem Blutpegel einstellende $CO_2$-Partialdruck $p_{CO2}$ entspricht dem mittels eines Blutgasanalysators bei gleicher Temperatur gemessenen $CO_2$-Partialdruck.

Die aktuell vorliegende Temperatur wird durch den in der Leitung (17) angeordneten Temperatursensor (8) bestimmt.

Ebenso wie die Temperatur ist für eine exakte Bestimmung des Partialdrucks auch der Normdruck zu berücksichtigen. Bei der Kompression des Gasgemisches auf einen Druck p ungleich dem Normaldruck $p_{norm}$ ergibt sich der $CO_2$-Partialdruck unter Normbedingungen zu

$$p_{CO_2}^{norm} = p_{CO_2}(p_{tot})\frac{p_{norm}}{p_{tot}} \qquad \textbf{Formel 4}$$

Der temperatur- und druckkompensierte $CO_2$-Partialdruck ($p_{CO2}(T, pnorm)$ im Blut wird dann mithilfe der Formeln 1 und 4 aus der $CO_2$-Konzentration im Gasraum über dem Blutpegel berechnet.

$$p_{CO_2}(T, p_{norm}) = \frac{1}{K_{H,CO_2}(T)}\frac{p_{norm}}{p_{tot}} c_{CO_2} \qquad \textbf{Formel 5}$$

Besonders vorteilhaft ist dabei die Verwendung der bei einer Hämodialyse üblichen Verbrauchsmaterialien für das extrakorporale Schlauchsystem. Um eine kontaktlose, weitgehend wartungsfreie und vor Kreuzkontamination sichere Messung zu ermöglichen, ist der Gassensor vorzugsweise in der Blutbehandlungsmaschine angeordnet.

Die Anordnung des Gassensors in der Blutbehandlungsmaschine und nicht direkt in der Ausgleichskammer bringt weiterhin den Vorteil mit sich, dass an den standardmäßig verwendeten Schlauchsystemen keine Änderungen erforderlich sind. Der Gassensor ist Teil der Hardware der Blutbehandlungsmaschine. In Abhängigkeit von der Distanz der Ausgleichskammer zu dem Gassensor, die sich maßgeblich aus der Länge der Schlauchverbindung (z.B. 10-30cm) ergibt, ist ein aktiver Transport der sich über dem Blutpegel einstellenden Gaskonzentration zu dem Gassensor notwendig.

Ohne Hilfsmittel, die eine Strömung vom Gasraum direkt über dem Blutpegel zum Messsensor erzeugen, ist der Transport der sich an der Blut/Luftkontaktstelle befindlichen Gaskonzentration, insbesondere der Kohlendioxidkonzentration, zum Gassensor alleine durch die Diffusion bestimmt. Für die eindimensionale Diffusion eines Gases in einen Raum, der das zu betrachtende Gas bisher nicht enthalten hat, gilt für das Konzentrationsmaximum der Diffusionsfront:

$$x_{\max}^2 = 2Dt_{\max}$$

Bei einer typischen Diffusionskonstanten $D \sim 1{,}6*10^{-5}$ $m^2/s$ benötigt das Gas für eine Diffusion von mehreren Zentimetern bereits mehrere Minuten (z.B. $x_{\max}$=20 cm mehr als 20 min).

In der in Figur 1 gezeigten erfindungsgemäßen Ausführungsform sind zu dem Zweck des Gastransports zu dem Gassensor (6) in der zweiten Leitung (17) verschiedene alternative Mittel, ein Ventil (14) bzw. eine zweite Blutpumpe (15) (gestrichelt dargestellt), gezeigt.

Beide, alternativ einzusetzende Mittel (14, 15) können von der Auswerte- und Steuereinheit (21) derart gesteuert werden, dass sie flussabwärts der Ausgleichskammer (1) eine Flussrestriktion erzeugen. Im Falle des Ventils (14) wird diese Flussrestriktion durch ein Schließen des Ventils erzeugt. Im Falle der zweiten Blutpumpe (15) wird die Flussrestriktion durch die Einstellung einer Förderrate der zweiten Blutpumpe (15), die geringer ist als die Förderrate der ersten Blutpumpe (4). Durch die Erzeugung einer Flussrestriktion in der zweiten Leitung (17) wird ein Heben des Flüssigkeitspegels in der Ausgleichskammer (1) von der Position (1a) auf (1b) erreicht. Dadurch wird Gas aus dem Gasraum der Ausgleichskammer (1) verdrängt und damit eine Durchströmung des Gassensors (6) erreicht. Um sicherzustellen, dass die sich in der Ausgleichskammer (1) einstellende Gaskonzentration den Gassensor (6) erreicht, sollte das verdrängte Volumen vorzugsweise mindestens doppelt so groß sein wie das sich in der Verbindung zwischen dem Anschluss (3a) des Verbindungsleitung (18) an der Ausgleichskammer (1) und dem Gassensor (6) befindliche Volumen. Bei für die Hämodialyse üblichen Blutschlauchsystemen reicht hierbei ein Hub von ca. 1-2cm.

Der sich beim Heben des Blutpegels aufbauende Luftdruck kann reduziert werden, indem entweder das Auslassventil (12) geöffnet und die Luft über Drosselventil (10) zur Umgebung entwichen gelassen wird, oder alternativ, indem nach Öffnen von Complianceventil (13) ein Druckausgleich in ein Compliancegefäß (9) (gestrichelt dargestellt) ermöglicht wird. Bei beiden alternativen Möglichkeiten wird ein Luftstrom durch den Gassensor (6) bewirkt, so dass an diesem nun die im Gasraum der Ausgleichskammer (1) vorherrschende Gaskonzentration gemessen werden kann.

Zur Rückstellung des Blutpegels (1b) auf (1a) wird Ventil (14) wieder geöffnet, bzw. die zweite Blutpumpe (15) mit einer höheren Förderrate gefahren. Ein Druckausgleich kann dann durch Einlassen von Umgebungsluft mittels eines Kompressors (11) bzw. über das Compliancegefäß (9) erfolgen.

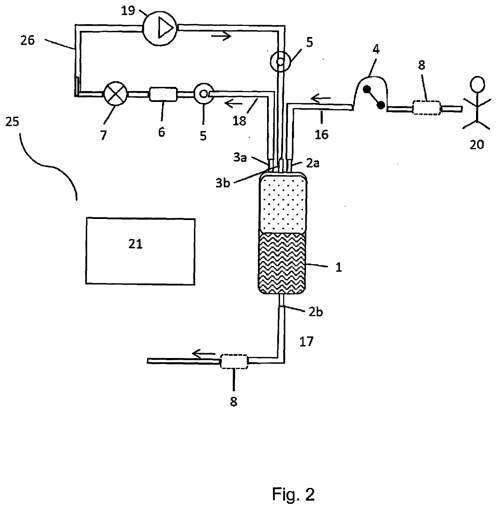

Alternativ kann bei der Verwendung einer wie in Abbildung 2 dargestellten Ausgleichskammer (1) eine kontinuierliche Durchströmung des Gassensors erreicht werden. Hierbei wird über dem Gasraum der Ausgleichskammer (1) mittels eines Gasfördermittels (19) eine Zirkulation von Anschluss (3a) über den Gassensor (6) über eine Rückführungsleitung (26) zurück zu Anschluss (3b) erreicht. Als Kontaminationsschutz sind hier in der Verbindungsleitung (18) und der Rückführungsleitung Hydrophobfilter (5) vorgesehen. Bei dem Gasfördermittel (19) kann es sich um Mittel beliebiger Art handeln, die eine geringe kontinuierliche Luftströmung erzeugen. Beispielsweise kann ein miniaturisierter Blasebalg eingesetzt werden, bei dem die Bewegung der Schenkel durch Piezo-Elemente erfolgt.

Die von dem Gassensor (6) aufgenommenen Messwerte werden von der Auswerteeinheit (21) ausgelesen und mittels Formel (5) wird der Partialdruck von Kohlendioxid in Blut bestimmt.

Dieser Partialdruck kann Hinweise auf unterschiedliche pathologische Zustände geben.

## Bestimmung des prädialytischen $p_{CO_2}$

Zu Beginn der Blutbehandlung gemessene Werte für den Partialdruck von Kohlendioxid unterscheiden sich nur wenig von dem prädialytischen Partialdruck. Ein niedriger Wert, insbesondere unterhalb des Normbereiches von 35-45 mmHg, hat meist eine respiratorisch kompensierte metabolische Azidose als Ursache. Somit kann bei der Messung eines $P_{CO_2}$, der unterhalb eines definierten Schwellenwerts liegt, vom Dialysegerät eine Warnung an den Anwender ausgegeben werden (Benutzerinterface/ Alarmierungseinheit (24)). Bei diesem Schwellenwert kann es sich um einen Absolutwert (z.B. klinischer Normbereich) oder um einen für diesen Patienten individuell bestimmten und in einer Speichereinheit hinterlegten Wert handeln, der z.B. aus dem Mittelwert der vergangenen Messungen für diesen Patienten bestimmt wird.

## Korrektur der metabolischen Azidose

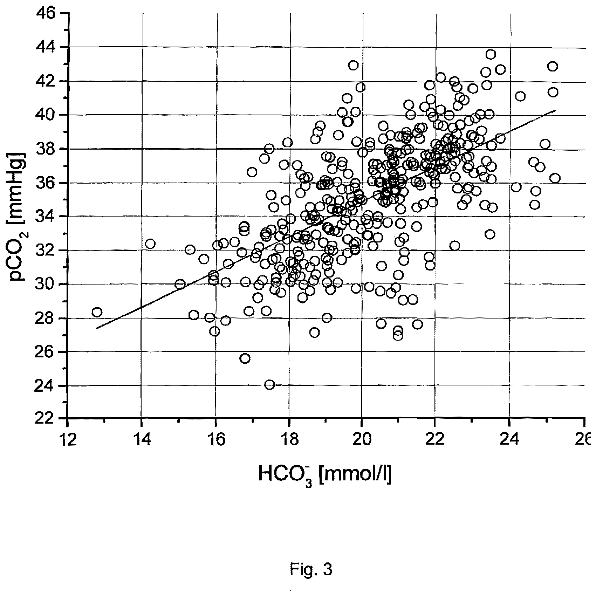

Die metabolische Azidose kann bei Dialysebehandlungen durch Zufuhr von Bicarbonat über die Dialyselösung korrigiert werden. In Figur 3 ist die Korrelation des prädialytischen $p_{CO_2}$ und der Bicarbonatkonzentration $HCO_3^-$ in Blut gezeigt. Nach einer Messung des $pCO_2$ Wertes kann durch die Dialysatzubereitungseinheit (23) des Dialysegerätes automatisiert die Einstellung der Bicarbonatkonzentration im Dialysat $c_D$ erfolgen. Hierzu kann ein 1-Pool-Modell verwenden werden, in welches das Verteilungvolumen V des Patienten, die mittels Online-Clearance-Messungen bestimmte Dialysedosis Kt/V, sowie die aus der $pCO_2$-Messung abgeschätzte Blut-Bicarbonatkonzentration $c_{B,O}$ eingehen.

$$c_B(t) = c_D + \left(c_{B,0} - c_D\right)e^{-\alpha\frac{Kt}{V}}$$ **Formel 6**

Der Faktor $\alpha$ berücksichtigt die gegenüber Harnstoff geringere Clearance von Bicarbonat und liegt bei -0,7.

$$c_{B,0} = f(p_{CO_2}) = a_0 + a_1 p_{CO_2}$$ **Formel 7**

Die Koeffizienten $a_0$ und $a_1$ können aus einer linearen Anpassung der in Figur 3 dargestellten Wertepaare erfolgen. Da die Stoffwechselvorgänge patientenabhängig sind, kann eine bessere Übereinstimmung zwischen dem abgeschätzten und realen Wert erreicht werden, wenn über einem gewissen Zeitraum mittels eines Referenzinstruments (Blutgasanalysators) $p_{CO_2}$ und Bicarbonat bestimmt und die Koeffizienten $a_0$ und $a_1$ patientenindividuell bestimmt werden.

Kann das zur Zeit $t_1$ erreichte Kt/V aufgrund der Dialyseeinstellungen (Blut-, Dialysat-, Substituatfluss, Dialysatorcharakteristik) abgeschätzt werden, so kann das zum Erreichen einer Blut-Bicarbonatkonzentration zur Zeit $t_1$ einzustellende Dialysat-Bicarbonat aus Formel 8 berechnet werden:

$$c_D = \frac{c_B(t_1) - c_{B,0}e^{-\alpha\frac{Kt}{V}}}{1 - e^{-\alpha\frac{Kt}{V}}}$$ **Formel 8**

Durch wiederholte Messungen des $p_{CO_2}$ sowie der bisher erreichten Dialysedosis ist auch eine Korrektur der Einstellung des Dialysat-Bicarbonats während der Dialyse möglich.

**Apnoe-Monitoring**

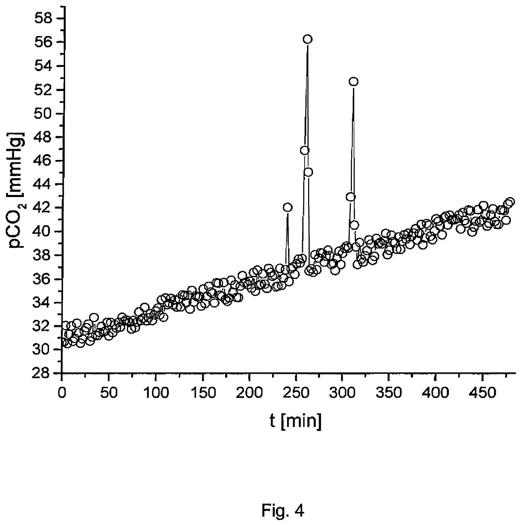

Wie eingangs bereits beschrieben, kann es gerade bei nächtlichen Langzeitdialysen aufgrund von Fehlern in den physiologischen Regelmechanismen oder durch partielle Obstruktion der Atemwege zu einer mangelhaften Atmung kommen. Dieses führt zu einem Anstieg des $p_{CO_2}$. Studien an Patienten mit obstruktiver Schlaf-Apnoe haben gezeigt, dass bei diesen der $p_{CO_2}$ beim Aufwachen am Morgen um >11 mmH höher liegt als vor dem Schlafen ("Changes in the arterial CO2 during a single nights sleep in patients with obstructive sleep apnea" Chin et al., Internal Medicine, Vol. 36, S. 454ff). Bei einem vorübergehenden Atemstillstand, der über eine Minute dauern kann, ist zu erwarten, dass der $p_{CO_2}$-Aanstieg deutlich höher ausfällt, wie der in Figur 4 gezeigte simulierte $p_{CO_2}$-Verlauf während einer Langzeitdialyse mit Apnoen zeigt. Gerade diese kritischen Atemaussetzer sind damit durch das beschriebene kontinuierliche $p_{CO_2}$-Monitoring erkennbar.

Bei einer Aufzeichnung des Verlaufs des $p_{CO_2}$ können Atemaussetzer diagnostiziert und bei der Therapieplanung berücksichtig werden.

Denkbar ist allerdings auch eine online-Überwachung zur Vermeidung längerer Atemaussetzer. So können in der Blutbehandlungsvorrichtung (25), bzw. Auswerteeinheit (21) unterschiedliche Warnkriterien hinterlegt sein, z.B. die Überschreitung einer absoluten Konzentrationsschwelle, Anstieg des $p_{CO_2}$ über eine Konzentrationsschwelle, die aus dem bisherigen Verlauf oder dem typischen Verlauf vorhergehenden Behandlungen berechnet wurde. Dabei können auch weitere Kriterien, wie z.B. ein bestimmte Dauer der Schwellenüberschreitung oder die Änderung im zeitlichen Verlauf des $p_{CO_2}$, z.B. stark beschleunigter Anstieg gegenüber dem bisherigen Verlauf, berücksichtigt werden.

Bei Auftreten dieser als kritisch bewerteten Zustände kann ein akustisches Signal erzeugt werden, das z.B. den Patienten oder das Pflegepersonal alarmiert.

Denkbar wäre auch ein Wecken oder die Stimulierung der Atmung durch Auslösen eines schwachen Elektoimpulses an dem Patienten.

Bei Verwendung einer Atemmaske kann eine Erhöhung des Beatmungsdrucks, bzw. des Luftstrome erfolgen.

**Diabetesmonitoring**

Mittels eines Acetonsensors (Massick et al., Proc. SPIE 6386, Optical Methods in the Life Sciences, 638600 (October 17, 2006)) können der Verlauf von Stoffwechselerkrankungen, z.B. Ketoazidose bei Diabetes, diagnostiziert und bei Aufzeichnung der Werte über mehrere Behandlungen ihr Verlauf beobachtet werden. Dieses kann hilfreich bei der Dosierung von Insulin bei Diabetikern an der Dialyse sein.

**Bestimmung der Dialyseeffizienz**

Zwischen dem Harnstoffkonzentration und der Ammoniumkonzentration im Atem besteht eine Korrelation ("Correlation of breath ammonia with blood urea nitrogen and creatinine during hemodialysis", Narasimhan et al., PNAS Vol. 98, S. 4617ff). Daher sind mittels eines Ammoniumsensors (z.B. "High sensitivity ammonia sensor using a hierarchical polyaniline/poly(ethylene-co-glycidyl methacrylate) nanofibrous composite membrane", Chen et al., ACS Appl Mater Interfaces, Vol. 24, S. 6473ff) praktisch alle Analysen möglich, die sonst auf der Bestimmung der Harnstoffkonzentration im Blut beruhen. Aus dem Verlauf der Ammoniumkonzentration zu Beginn aufeinanderfolgender Dialysen kann daher auf den Proteinumsatz (PCR = protein catabolic rate) geschlossen werden. Aus der intradialytischen Änderung der Ammoniumkonzentration kann die Dialyseeffizienz Kt/V berechnet werden:

$$\frac{Kt}{V} = -\ln \frac{c_{NH_3}(t)}{c_{NH_3}(0)} \qquad \textbf{Formel 9}$$

Im Gegensatz zu Messungen auf der Dialysatseite ist hier keine Korrektur der Messwerte bzgl. der Flussraten nötig (cf. BBraun: Adimea).

**Funktionstest des Hydrophobfilters**

In einer Ausführungsform der Erfindung kann in der Blutbehandlungsvorrichtung (25) neben dem Gassensor (6) auch der Drucksensor (7) zur Überwachung des Druckes in dem Blutschlauchsystem vorgesehen sein, so dass der Verbindungsschlauch (18) sowohl zu dem Gassensor (6) als auch zu dem Drucksensor (7) führt. Die Messdaten des Gassensors (6) können dann auch zur Durchführung eines Funktionstests des Hydrophofilters (5) verwendet werden. Bei Verwendung einer Vorrichtung wie in Figur 1 gezeigt, bei der ein Druckausausgleich zur Umgebung erfolgt, kann die Durchlässigkeit des Hydrophobfilters (5) mittels des Gassensors (6) und der Auswerteeinheit (21) getestet werden. Durch eine Benetzung des Hydrophobfilters (5) mit Blut oder Dialysierflüssigkeit wird dieser auch für Gas undurchlässig, so dass keine verlässliche Druckmessung mehr möglich ist. Da die sich aufgrund des Blut-$p_{CO2}$ einstellende $CO_2$-Konzentration im Gasraum von (1) ca. 5% beträgt, der normale $CO_2$-Gehalt der Umgebungsluft aber nur ca. 400ppm=0,004%, kann beim Heben und Senken de Pegels in der Ausgleichskammer (1) festgestellt werden, ob eine Gasverbindung zwischen der Ausgleichskammer (1) und dem Gassensor (6) besteht. Bei einem blutgefüllten Schlauchsystem ist beim Heben des Pegels mit einem Anstieg der $CO_2$-Konzentration auf >1% zu rechnen, beim Senken mit einem Abfall auf unter 0,1%. Bleibt dieses aus, so kann auf einen blockierten Hydrophobfilter (5) geschlossen werden.

Die hier beschriebenen Ausführungsformen bieten die Möglichkeit, während einer extrakorporalen Blutbehandlung die Konzentration in Blut gelöster Gase ohne die Verwendung zusätzlicher Sensoren oder Blutentnahmen und -analysen zu bestimmen und zu überwachen. Die Messung der Gaskonzentration kann unter Verwendung der üblichen Blutschlauchsysteme, kontaktfrei und kontaminationslos erfolgen, so dass für den Patienten keine zusätzlichen Belastungen oder Risiken entstehen. Die Messwerte können zur Beobachtung von Vitalparameter der Patienten, zur Bestimmung der Behandlungseffizienz oder als Funktionstest für Komponenten des extrakorporalen Kreislaufes verwendet werden.

**Liste der Bezugszeichen**

Ausgleichskammer (1)
Flüssigkeitspegel (1a)
Flüssigkeitspegel (1b)
Anschluss Kammer (2a), (2b)
Anschluss Kammer (3a)
Blutpumpe (4)
Hydrophobfilter (5)
Gassensor (6)

Drucksensor (7)
Temperatursensor (8)
Compliancegefäß (9)
Drosselventil (10)
Kompressor (11)
Auslassventil (12)
Complianceventil (13)
Ventil (14)
Blutpumpe (15)
Arterieller Teil des Blutschlauchsystem (16)
Venöser Teil des Blutschlauchsystem (17)
Verbindungsschlauch. (18)
Gasfördermittel (19)
Patient (20)
Auswerteeinheit (21)
Blutreinigungseinheit (22)
Dialysatzubereitungseinheit (23)
Benutzerinterface/ Alarmierungseinheit (24)
Blutbehandlungsvorrichtung (25)
Rückführungsleitung (26)


**Patentansprüche**

1.  Blutbehandlungsvorrichtung (25) mit einem extrakorporalen Blutkreislauf umfassend eine erste Leitung (16), an einem Ende zur Blutentnahme verbindbar mit einem Patienten (20) und am anderen Ende verbunden mit einer Ausgleichskammer (1), eine erste Blutpumpe (4) zur Förderung von Blut im extrakorporalen Kreislauf in die Ausgleichskammer (1), wodurch in dieser ein Blutpegel (1a, 1b) entsteht, und ein Kontakt des Blutes zu einem sich oberhalb des Blutpegels befindlichen Gasraums gebildet wird, und einer zweiten Leitung (17) zur Weiterleitung des Blutes von der Ausgleichskammer (1) zu einer Blutreinigungseinheit (22), **dadurch gekennzeichnet, dass** die Blutbehandlungsvorrichtung (25) einen mit der Ausgleichskammer (1) verbindbaren Gassensor (6) zur Messung der Konzentration eines Gases in dem Gasraum und eine Auswerteeinheit (21) zum Auslesen und zur Auswertung der Messdaten des Gassensors (6) umfasst.

2.  Blutbehandlungsvorrichtung (25) nach Anspruch 1 **dadurch gekennzeichnet, dass** der Gassensor (6) ein Sensor für Kohlendioxid oder Ammonium oder Aceton ist.

3.  Blutbehandlungsvorrichtung (25) nach Anspruch 1 und/oder 2 **dadurch gekennzeichnet, dass** der Gassensor (6) beheizbar ist.

4.  Blutbehandlungsvorrichtung (25) nach einem der vorangehenden Ansprüche, **dadurch gekennzeichnet, dass** in der ersten Leitung (16), der zweiten Leitung (17) oder der Ausgleichskammer (1) des extrakorporalen Blutkreislaufs ein Temperatursensor (8) zur Bestimmung eines Messwerts der Bluttemperatur angeordnet ist, wobei die Auswerteeinheit (21) ausgelegt ist den Messwert zur Temperaturkompensation zu berücksichtigen.

5.  Blutbehandlungsvorrichtung (25) nach einem der vorangehenden Ansprüche **dadurch gekennzeichnet, dass** sie einen Drucksensor (7) aufweist zur Bestimmung eines Messwerts für den Druck in der Ausgleichskammer (1), wobei die Auswerteeinheit (21) ausgelegt ist den Messwert zur Druckkompensation zu berücksichtigen.

6.  Blutbehandlungsvorrichtung (25) nach einem der vorangehenden Ansprüche **dadurch gekennzeichnet, dass** die Ausgleichskammer (1) mittels zumindest einer Verbindungsleitung (18), vorzugsweise einem Verbindungsschlauch (18), mit dem Gassensor (6) verbunden ist.

7.  Blutbehandlungsvorrichtung (25) nach Anspruch 6 **dadurch gekennzeichnet, dass** in der Verbindungsleitung (18) zwischen dem Gassensor (6) und der Ausgleichskammer (1) ein Hydrophobfilter (5) angeordnet ist.

8.  Blutbehandlungsvorrichtung (25) nach einem der vorangehenden Ansprüche **dadurch gekennzeichnet, dass** sie Mittel zur Erzeugung einer Gasströmung aus der Ausgleichskammer (1) durch die Verbindungsleitung (18) zu dem Gassensor (6) aufweist und das die Auswerteeinheit als Steuer- und Auswerteeinheit (21) ausgebildet ist.

9. Blutbehandlungsvorrichtung (25) nach Anspruch 8 **dadurch gekennzeichnet, dass** das Mittel zur Erzeugung der Gasströmung als Ventil (14) oder als zweite Blutpumpe (15) in der zweiten Leitung (17) des extrakorporalen Blutkreislaufs ausgebildet ist, wobei das Ventil (14) oder die zweite Blutpumpe (15) von der Steuer- und Auswerteeinheit (21) so angesteuert werden, dass in der zweiten Leitung (17) zeitweise eine geringere Blutflussrate herrscht als vor der Ausgleichskammer(1) in der ersten Leitung (16), wodurch eine Verschiebung des Blutpegels (1a) in der Ausgleichskammer (1) erzeugt werden kann.

10. Blutbehandlungsvorrichtung (25) nach Anspruch 9 **dadurch gekennzeichnet, dass** sie Ausgleichsmittel (9, 10, 11 12, 13) für eine bei einer Blutpegelverschiebung auftretenden Druckerhöhung aufweist.

11. Blutbehandlungsvorrichtung (25) nach Anspruch 8 **dadurch gekennzeichnet, dass** der Gassensor (6) und die Ausgleichskammer (1) weiterhin mit einer Rückführungsleitung (26) verbunden sind, wobei in dieser Rückführungsleitung (26) ein Gasfördermittel (19) angeordnet ist, dass zwischen Gassensor (6) und Ausgleichskammer (1) ein zirkulierender Gasstroms entsteht.

12. Blutbehandlungsvorrichtung (25) nach Anspruch 1 **dadurch gekennzeichnet, dass** die Blutreinigungseinheit (22) ein Dialysefilter oder ein Hämofilter oder ein Plasmafilter ist.

13. Blutbehandlungsvorrichtung (25) nach Anspruch 1 **dadurch gekennzeichnet, dass** die Auswerteeinheit (21) dazu ausgelegt ist den Partialdruck eines Gases in Blut zu bestimmen und in der Auswerteeinheit (21) ein Sollbereich für den Partialdruck des Gases hinterlegt ist und im Falle eines gemessenen Partialdrucks außerhalb des Sollbereichs ein Signal an eine Alarmeinrichtung (24) weiterleitet, die dann ein Alarmsignal ausgibt.

14. Blutbehandlungsvorrichtung (25) nach Anspruch 1 **dadurch gekennzeichnet, dass** sie eine Dialysatzubereitungseinheit (23) zur Versorgung der Blutreinigungseinheit (22) mit Dialysat aufweist, und die Auswerteinheit (21) als Auswerte- und Steuereinheit ausgebildet ist um aus den Messwerten des Gassensors (6) einen Partialdruck eines Gases zu bestimmen und dem Partialdruck des Gases eine Zusammensetzung des Dialysates zuzuordnen, die dann von der Dialysatzubereitungseinheit (23) bereitgestellt wird

15. Verfahren zur Bestimmung des Partialdrucks eines Gases in Blut des extrakorporalen Blutkreislauf einer Blutbehandlungsvorrichtung (25) nach Anspruch 1 **dadurch gekennzeichnet, dass** die Konzentration des Gases mittels eines Gassensors (6) im Gasraum der Ausgleichskammer (1) gemessen wird und aus diesen Messdaten durch die Auswerteeinheit (21) der Partialdruck des Gases im Blut berechnet wird.

16. Verfahren zur Bestimmung des Partialdrucks eines Gases in Blut nach Anspruch 15 **dadurch gekennzeichnet, dass** in der Blutbehandlungsvorrichtung (25) eine Gasströmung von der Ausgleichskammer(1) zu dem Gassensor (6) erzeugt wird.

## Claims

1. A blood treatment device (25) having an extracorporeal blood circulation, comprising a first line (16) that can be connected to a patient (20) at one end for withdrawing blood and connected to an equalizing chamber (1) at the other end, a first blood pump (4) for delivering blood in the extracorporeal circulation to the equalizing chamber (1), so that a blood level (1a, 1b) develops therein, and contact of the blood with a gas space situated above the blood level is formed, and a second line (17) for conveying the blood from the equalizing chamber (1) to a blood purification unit (22), **characterized in that** the blood treatment device (25) comprises a gas sensor (6) that can be connected to the equalizing chamber (1) for measuring the concentration of a gas in the gas space, and an evaluation unit (21) for readout and evaluation of the measurement data of the gas sensor (6) .

2. The blood treatment device (25) according to claim 1, **characterized in that** the gas sensor (6) is a sensor for carbon dioxide or ammonium or acetone.

3. The blood treatment device (25) according to claim 1 and/or 2, **characterized in that** the gas sensor (6) can be heated.

4. The blood treatment device (25) according to any one of the preceding claims, **characterized in that** a temperature sensor (8) for determining a measured value of the blood temperature is arranged in the first line (16), the second line (17) or the equalizing chamber (1) of the extracorporeal blood circulation, wherein the evaluation unit (21) is designed to take into account the measured value for temperature compensation.

5. The blood treatment device (25) according to any one of the preceding claims, **characterized in that** it has a pressure sensor (7) for determining a measured value for the pressure in the equalizing chamber (1), wherein the evaluation unit (21) is designed to take into account the measured value for pressure compensation.

6. The blood treatment device (25) according to any one of the preceding claims, **characterized in that** the equalizing chamber (1) is connected to the gas sensor (6) by means of at least one connecting line (18), preferably a connecting tubing (18).

7. The blood treatment device (25) according to claim 6, **characterized in that** a hydrophobic filter (5) is arranged between the gas sensor (6) and the equalizing chamber (1) in the connecting line (18).

8. The blood treatment device (25) according to any one of the preceding claims, **characterized in that** it has means for generating a gas stream from the equalizing chamber (1) through the connecting line (18) to the gas sensor (6), and the evaluation unit is designed as a control and evaluation unit (21).

9. The blood treatment device (25) according to claim 8, **characterized in that** the means for generating the gas stream is designed as a valve (14) or as a second blood pump (15) in the second line (17) of the extracorporeal blood circulation, wherein the valve (14) or the second blood pump (15) is controlled by the control and evaluation unit (21), so that a lower blood flow rate prevails temporarily in the second line (17) than in the first line (16) upstream from the equalizing chamber (1), so that a shift in the blood level (1a) in the equalizing chamber (1) can be created.

10. The blood treatment device (25) according to claim 9, **characterized in that** it has equalizing means (9, 10, 11, 12, 13) for an increase in pressure occurring with a shift in blood level.

11. The blood treatment device (25) according to claim 8, **characterized in that** the gas sensor (6) and the equalizing chamber (1) are still connected to a return line (26), wherein a gas delivery means (19) is arranged in this return line (26), such that a circulating gas stream is formed between the gas sensor (6) and the equalizing chamber (1).

12. The blood treatment device (25) according to claim 1, **characterized in that** the blood purification unit (22) is a dialysis filter or a hemofilter or a plasma filter.

13. The blood treatment device (25) according to claim 1, **characterized in that** the evaluation unit (21) is designed to determine the partial pressure of a gas in blood, and an ideal range for the partial pressure of the gas is stored in the evaluation unit (21), and in the case of a measured partial pressure outside of the ideal range, a signal is relayed to an alarm device (24), which then outputs an alarm signal.

14. The blood treatment device (25) according to claim 1, **characterized in that** it has a dialysate preparation unit (23) for supplying the blood purification unit (22) with dialysate, and the evaluation unit (21) is designed as an evaluation and control unit to determine a partial pressure of a gas from the measured values of the gas sensor (6) and to assign a composition of the dialysate to the partial pressure of the gas, said composition then being supplied by the dialysate preparation unit (23).

15. A method for determining the partial pressure of a gas in the blood of the extracorporeal blood circulation of a blood treatment device (25) according to claim 1, **characterized in that** the concentration of the gas is measured by means of a gas sensor (6) in the gas space of the equalizing chamber (1), and the partial pressure of the gas in the blood is calculated from the measured data by the evaluation unit (21).

16. The method for determining the partial pressure of a gas in blood according to claim 15, **characterized in that** a gas stream from the equalizing chamber (1) to the gas sensor (6) is created in the blood treatment device (25).

**Revendications**

1. Dispositif de traitement sanguin (25) comportant un circuit sanguin extracorporel comprenant une première conduite (16) pouvant être connectée par une extrémité pour prélèvement à un patient (20) et connectée par une autre extrémité à une chambre de compensation (1), une première pompe hémostatique (4) pour transporter du sang dans le circuit extracorporel dans la chambre de compensation (1), ce qui établit dans celle-ci un niveau sanguin (1a, 1b), et un contact du sang avec un espace gazeux se trouvant au-dessus du niveau sanguin étant établi, et une seconde conduite (17) pour le transfert du sang depuis la chambre de compensation (1) vers une unité d'épuration de sang (22), **caractérisé en ce que** le dispositif de traitement sanguin (25) comprend un capteur de gaz (6) connectable à la chambre de compensation (1) pour mesurer la concentration d'un gaz dans l'espace gazeux et une unité d'exploitation (21) pour lire et pour exploiter les données de mesure du capteur de gaz (6).

2. Dispositif de traitement sanguin (25) selon la revendication 1, **caractérisé en ce que** le capteur de gaz (6) est un capteur de dioxyde de carbone ou d'ammonium ou d'acétone.

3. Dispositif de traitement sanguin (25) selon la revendication 1 et/ou 2, **caractérisé en ce que** le capteur de gaz (6) peut être chauffé.

4. Dispositif de traitement sanguin (25) selon une des revendications précédentes, **caractérisé en ce que**, dans la première conduite (16), la seconde conduite (17) ou la chambre de compensation (1) du circuit sanguin extracorporel, un capteur de température (8) destiné à définir une valeur de mesure de la température du sang est disposé, l'unité d'exploitation (21) étant conçue pour tenir compte de la valeur de mesure pour la compensation de température.

5. Dispositif de traitement sanguin (25) selon une des revendications précédentes, **caractérisé en ce qu'**il présente un capteur de pression (7) pour la définition d'une valeur de mesure pour la pression dans la chambre de compensation (1), l'unité d'exploitation (21) étant conçue pour tenir compte de la valeur de mesure pour la compensation de pression.

6. Dispositif de traitement sanguin (25) selon une des revendications précédentes, **caractérisé en ce que** la chambre de compensation (1) est connectée au moyen d'au moins une conduite de raccordement (18), de préférence un tuyau de raccordement (18), au capteur de gaz (6).

7. Dispositif de traitement sanguin (25) selon la revendication 6, **caractérisé en ce que**, dans la conduite de raccordement (18), entre le capteur de gaz (6) et la chambre de compensation (1), un filtre hydrophobe (5) est disposé.

8. Dispositif de traitement sanguin (25) selon une des revendications précédentes, **caractérisé en ce qu'**il présente des moyens de génération d'un flux gazeux depuis la chambre de compensation (1) à travers la conduite de raccordement (18) jusqu'au capteur de gaz (6) et que l'unité d'exploitation est réalisée sous forme d'une unité de commande et d'exploitation (21).

9. Dispositif de traitement sanguin (25) selon la revendication 8, **caractérisé en ce que** les moyens de génération du flux gazeux se présentent sous forme d'une soupape (14) ou d'une seconde pompe hémostatique (15) dans la seconde conduite (17) du circuit sanguin extracorporel, la soupape (14) ou la seconde pompe hémostatique (15) étant commandée par l'unité de commande et d'exploitation (21) de manière à ce que règne provisoirement dans la seconde conduite (17) un débit sanguin plus faible qu'en amont de la chambre de compensation (1) dans la première conduite (16), ce qui permet de générer un décalage du niveau sanguin (1a) dans la chambre de compensation (1).

10. Dispositif de traitement sanguin (25) selon la revendication 9, **caractérisé en ce qu'**il présente des moyens de compensation (9, 10, 11, 12, 13) pour une hausse de pression survenant en cas de décalage du niveau sanguin.

11. Dispositif de traitement sanguin (25) selon la revendication 8, **caractérisé en ce que** le capteur de gaz (6) et la chambre de compensation (1) sont en outre connectés à une conduite de renvoi (26), un moyen de transport de gaz (19) étant disposé dans cette conduite de renvoi (26), de sorte qu'un flux gazeux circulant se crée entre le capteur de gaz (6) et la chambre de compensation (1).

12. Dispositif de traitement sanguin (25) selon la revendication 1, **caractérisé en ce que** l'unité d'épuration de sang (22) est un filtre de dialyse ou un hémofiltre ou un filtre plasmatique.

13. Dispositif de traitement sanguin (25) selon la revendication 1, **caractérisé en ce que** l'unité d'exploitation (21) est conçue pour définir la pression partielle d'un gaz dans le sang et qu'une plage théorique pour la pression partielle du gaz est enregistrée dans l'unité d'exploitation (21) et que, dans le cas d'une pression partielle mesurée se situant en dehors de la plage théorique, elle envoie un signal à un dispositif d'alarme (24) qui émet ensuite un signal d'alarme.

14. Dispositif de traitement sanguin (25) selon la revendication 1, **caractérisé en ce qu'**il présente une unité de préparation de dialysat (23) pour alimenter l'unité d'épuration de sang (22) en dyalisat et que l'unité d'exploitation (21) est réalisée sous forme d'une unité d'exploitation et de commande pour déterminer une pression partielle d'un gaz à partir des valeurs de mesure du capteur de gaz (6) et associer à la pression partielle du gaz une composition du dialysat qui est ensuite préparé par l'unité de préparation de dialysat (23) .

15. Procédé de définition de la pression partielle d'un gaz dans le sang du circuit sanguin extracorporel d'un dispositif de traitement sanguin (25) selon la revendication 1, **caractérisé en ce que** la concentration du gaz est mesurée au moyen d'un capteur de gaz (6) dans l'espace gazeux de la chambre de compensation (1) et que, à partir de ces données de mesure, la pression partielle du gaz dans le sang est calculée par l'unité d'exploitation (21).

16. Procédé de définition de la pression partielle d'un gaz dans le sang selon la revendication 15, **caractérisé en ce que**, dans le dispositif de traitement sanguin (25), un flux de gaz est généré de la chambre de compensation (1) vers le capteur de gaz (6).

Fig. 1

Fig. 2

Fig. 3

Fig. 4

**IN DER BESCHREIBUNG AUFGEFÜHRTE DOKUMENTE**

**In der Beschreibung aufgeführte Patentdokumente**

- WO 2013156435 A

**In der Beschreibung aufgeführte Nicht-Patentliteratur**

- **KELLY PASCHKE et al.** Potential Application of Exhaled Breath Monitoring in Renal Replacement Therapy. *Poster Presentation ASN,* 2013
- **NARASHIMHAN et al.** correlation of breath ammonia with blood urea nitrogen and creatinine during hemodialysis. *PNAS,* vol. 98, 4617ff
- **CHIN et al.** Changes in the arterial CO2 during a single nights sleep in patients with obstructive sleep apnea. *Internal Medicine,* vol. 36, 454ff
- **MASSICK et al.** Optical Methods in the Life Sciences. *Proc. SPIE 6386,* 17. Oktober 2006, vol. 638600
- **NARASIMHAN et al.** Correlation of breath ammonia with blood urea nitrogen and creatinine during hemodialysis. *PNAS,* vol. 98, 4617ff
- **CHEN et al.** High sensitivity ammonia sensor using a hierarchical polyaniline/poly(ethylene-co-glycidyl methacrylate) nanofibrous composite membrane. *ACS Appl Mater Interfaces,* vol. 24, 6473ff